# EUROPÄISCHE PATENTSCHRIFT

(11) **EP 0 325 691 B1**
(45) Veröffentlichungstag und Bekanntmachung des Hinweises auf die Patenterteilung: **07.04.1993**
(21) Anmeldenummer: 88115908.1
(22) Anmeldetag: 27.09.1988
(51) Int. Cl.: C07K 3/08, C07K 3/22

(54) **Verfahren zur Solubilisierung von unlöslichem Protein**
Process for the solubilization of insoluble protein
Procédé de solubilisation des protéines insolubles

(30) Priorität: 25.01.1988 DE 3802045
(43) Veröffentlichungstag der Anmeldung: 02.08.1989
(73) Patentinhaber: Consortium für elektrochemische Industrie GmbH, D-81379 München (DE)
(72) Erfinder: Fanning, Ellen, Prof. Dr., D-8000 München 19 (DE); Höss, Adolf, Dipl.-Chem., D-8164 Hausham (DE); Arthur, Avril, Dr., D-8000 München 40 (DE)

(56) Entgegenhaltungen:
- PROTEIN PURIFICATION, Micro to Marco, p 429-442
- BIOTECHNOLOGY 6 S. 1214-17
- BIOFUTUR 77, p. 3-9

## Beschreibung

Die Erfindung betrifft ein Verfahren zur Solubilisierung von unlöslichem Protein, das von kultivierten Zellen exprimiert wird.

Kultivierte Zellen, insbesondere Bakterien, vor allem Escherichia coli, werden technisch zur Überproduktion von Proteinen herangezogen, insbesondere von Proteinen, die von klonierten Genen anderer Organismen kodiert werden. In vielen Fällen ist die Überexpression von kommerziell interessanten Proteinen befriedigend gelungen, wie Interferon-β und Insulin als Beispiele belegen. Es hat sich jedoch gezeigt, daß die Überproduktion infolge Unlöslichkeit überexprimierter Proteine problematisch sein kann. Für E. coli findet sich eine Übersicht in Biotechnology, 5 (1987) 883-890. Die Proteine werden als unter dem Elektronenmikroskop sichtbare Einschlußkörper abgelagert; vgl. beispielsweise Science, 215 (1982) 687-689. Zwar bieten diese Einschlußkörper den Vorteil, daß die rekombinanten Proteine weitgehend vor Proteolyse geschützt sind; vgl. beispielsweise EMBO Journal, 3 (1984) 1429-1434. Die Isolierung von rekombinanten Proteinen in solubilisierter oder aktiver Form aus diesen Einschlußkörpern bzw. Ablagerungen ist jedoch mit großen Schwierigkeiten verbunden. Oft werden Harnstoff, Natriumdodecylsulfat (SDS) oder andere Denaturierungsmittel zur Auflösung der Proteine eingesetzt. Diese Detergentien führen jedoch zu einer unerwünschten Denaturierung der Proteine; vgl. beispielsweise folgende Übersichten: Marston (1987), The purification of eukaryotic polypeptides expressed in Escherichia coli. In: DNA Cloning, Vol. III, ed. D. Glover, Seiten 59-88, IRL Press, Oxford; PNAS, 80 (1983) 906-910 oder PNAS, 82 (1985) 2354-2358. Eine Renaturierung ist jedoch mit Verlusten an aktivem Material verbunden, vor allem weil die Renaturierung in der Regel nicht vollständig ist. Es gibt auch Fälle, in denen die Renaturierung nicht mehr möglich ist, so daß man auf das unlösliche Protein verzichten muß und nur auf den löslichen Anteil als Proteinreservoir zurückgreifen kann; vgl. beispielsweise Biotechnology, 5 (1987) 960-965.

Die Aufgabe, unlösliche Proteine, die durch kultivierte Zellen exprimiert worden sind, einer Solubilisierung zu unterwerfen, wird nun erfindungsgemäß durch ein Verfahren gelöst, bei dem man das unlösliche Protein mit einem Inonenaustauscher zusammenbringt und danach wieder von ihm trennt.

Die wesentlichen Vorteile des erfindungsgemäßen Vergehens sind darin zu sehen, daß auf Denaturierungsmittel bzw. Detergentien verzichtet werden kann, das erfindungsgemäße Verfahren leicht und schnell durchgeführt werden kann und eine nahezu quantitative Ausbeute an solubilisiertem Protein resultiert.

Es ist zwar bereits bekannt, Proteine - nach Abzentrifugieren von unlöslichem Material - an Ionenaustauschern zu adsorbieren, jedoch ist bei dieser bekannten Methode - was nicht überrascht - nur lösliches Protein eingesetzt worden; vgl. beispielsweise Current Protocols in Molecular Biology, 1987, Seite 10.9.2, New York (Greene Publ. Ass. and Wiley-Interscience). Dieser Stand der Technik ist insofern von Bedeutung, als der Fachmann mit der Auswahl geeigneter Ionenaustauscher beim Einsatz eines bestimmten Proteins vertraut ist.

Es ist naheliegend, das erfindungsgemäße Verfahren auf Proteine anzuwenden, die von Bakterien exprimiert werden, die man technisch kultiviert, wie vor allem E. coli. Das erfindungsgemäße Verfahren läßt sich jedoch auch auf Proteine anwenden, die von eukaryotischen Zellen exprimiert werden, die technisch in Gewebekulturen gehalten werden, wie beispielsweise Insektenzellen; vgl. beispielsweise PNAS, 84 (1987) 5700-5804 oder Genetic Engineering, 8 (1986) 277-298.

Bei der Durchführung des erfindungsgemäßen Verfahrens kann man das unlösliche Protein mit dem Ionenaustauscher in der Kulturbrühe in Gegenwart der aufgeschlossenen Zellen und der Zelltrümmer zusammenbringen. Natürlich kann man das unlösliche Protein mit dem Ionenaustauscher auch erst nach Abtrennung der aufgeschlossenen Zellen und der Zelltrümmer zusammenbringen.

Zur Wahl eines geeigneten Ionenaustauschers für ein bestimmtes Protein sind dem Fachmann bei der Einfachheit des beanspruchten Verfahrens routinemäßige Eignungsversuche zuzumuten. Darüberhinaus wird der vorstehend genannte Stand der Technik dem Fachmann eine Orientierungshilfe geben. Bei dem Ionenaustauscher kann die Matrix organischer Natur oder ein organisches Polymeres oder ein Polysaccharid sein, beispielsweise Agarose. Beispiele für erfindungsgemäß einsetzbare Ionenaustauscher sind Sepharosen. Ein spezielles Beispiel für einen Ionenaustauscher, dessen Einsatz nicht nur auf die Solubilisierung eines einzigen speziellen Proteins beschränkt ist, ist Q-Sepharose® (Q-Sepharose-Fast-Flow® von Pharmacia). Q-Sepharose® ist ein Beispiel für einen Anionenaustauscher. Derartiger Anionenaustauscher wird man sich bedienen, wenn man im basischen Milieu arbeitet. Jedoch ist auch die Anwendung von Kationenaustauschern möglich, wenn man ein saueres Milieu vorsieht. Als Beispiel sei S-Sepharose® (S-Sepharose-Fast-Flow® von Pharmacia) genannt.

Verwendet man beispielsweise Bakterienzellen zur Überproduktion eines bestimmten Proteins, so kann man die Zellen in bekannter Weise aufschließen, beispielsweise durch Ultraschall, Lysozym, osmotischen Schock, Einfrieren/Auftauen oder eine Kombination dieser Maßnahmen. Zur Schonung des Proteins wird das flüssige Medium vorzugsweise gepuffert. Arbeitet man in größerem Maßstab, so ist es vorteilhaft, das Ionenaustauscher-Material dem gepufferten Kulturmedium zuzugeben, ohne die aufgeschlossenen Zellen und die Zelltrümmer abzutrennen. Wird das Ionenaustauscher-Material in körniger Form zugegeben, so kann man es nach einiger Zeit, etwa nach 1 bis 2 h, vom Kulturmedium abfiltrieren oder abzentrifugieren. Gegebenenfalls muß adsorbiertes Protein vom Ionenaustauscher eluiert werden, beispielsweise durch Zugabe eines salzhaltigen Puffers. Die Abtrennung des Ionenaustauschers vom proteinhaltigen flüssigen Medium kann wiederum durch Abfiltrieren oder Abzentrifugieren erfolgen. Das proteinhaltige flüssige Medium läßt sich in bekannter Weise zur Gewinnung oder Verwertung des Proteins aufarbeiten.

Nachstehend wird die Erfindung anhand von 8 Beispielen und einer Figur näher erläutert.

### Beispiel 1: Solubilisierung vom SV40-T-Antigen-Derivat Th (Peptid Th)

Man induzierte 5 ml über Nacht kultivierter E.-coli-Zellen (JM 103), die das Plasmid pTh enthielten (siehe Fig.) bei 37 °C in 100 ml L-Broth-Medium 4 h lang mit 5 mM Isopropyl-ß-D-thiogalactopyranosid (IPTG). Danach zentrifugierte man mit einer Zentrifuge (Sorvall Rotor SS34) 5 min lang bei 8000 U/min. Der überstand, das L-Broth-Medium, wurde abgegossen und das Pellet in 2,5 ml Puffer A resuspendiert (Puffer A: 50 mM Tris-HCl (pH 8,0), 50 mM NaCl, 1 mM EDTA, 1 mM DTT, 1 mM PMSF und 10 Glycerin). Um die Zellen aufzuschließen, gab man zur Suspension Lysozym (5 mg/ml), hielt die Suspension 15 min lang auf Eis, beschallte 3 x 20 sec lang mit Ultraschall und gab wieder 15 min lang auf Eis. Das Lysat wurde zum gleichen Volumen Q-Sepharose-Fast-Flow® (Q-Sepharose-FF®) gegeben, wobei im Puffer A equilibriert wurde. Danach wurde das System 2 h lang bei 4 °C auf einem Rotator geschüttelt, wonach 15 min lang bei 5000 U/min zentrifugiert wurde. Der Überstand wurde verworfen. Der Niederschlag wurde im gleichen Volumen Puffer B aufgenommen (Puffer B: Puffer A, jedoch mit 250 mM NaCl-Konzentration). Danach wurde 15 min lang bei 4 °C auf einem Rotator geschüttelt und schließlich 15 min lang bei 5000 U/min abzentrifugiert. Der Überstand wurde zur Aktivitätsbestimmung von Th in einen DNA-Bindungstest eingesetzt. Das Ergebnis ist in Tabelle 1 gezeigt.

**Tabelle 1**

| DNA-Bindungstest von Th-(Isolierung nach Beispiel 1) | | | | |
|---|---|---|---|---|
| Fraktion | Volumen ( µl ) | Densitometer Messung (%) | Th-Konz. ( µg ) | DNA-Bindungsaktivität (%) |
| Gesamtlysat | 500 | 5,6 | 2 | 100 |
| Überstand der Bindung an Q-Sepharose® | 500 | 0,0 | 0 | 0 |
| Eluat des Lysats von Q-Sepharose® | 500 | 16,7 | 6 | 298 |

### Beispiel 2: Solubilisierung von SV40-T-Antigen (Th)

Es wurde Beispiel 1 mit der Ausnahme wiederholt, daß nach dem Aufschluß der Zellen 15 min lang bei 4 °C zentrifugiert wurde, wobei einmal der Überstand und das andere Mal der im Puffer A resuspendierte Niederschlag mit dem Ionenaustauscher behandelt wurden. Die Aktivität des renaturierten Proteins wurde wie in Beispiel 1 bestätigt (siehe Tabelle 2).

**Tabelle 2**

| DNA-Bindungstests von Th-(Isolierung nach Beispiel 2) | | | | |
|---|---|---|---|---|
| Fraktion | Volumen ( µl ) | Densitometer Messung (%) | Th-Konz. ( µg ) | DNA-Bindungsaktivität (%) |
| Gesamtlysat | 500 | 5,6 | 2 | 100 |
| Eluat des Überstandes von Q-Sepharose® | 500 | 5,8 | 2 | 99 |
| Eluat des Pellets von Q-Sepharose® | 500 | 10,9 | 4 | 194 |

### Beispiel 3: Solubilisierung von Interleukin-2 (IL-2)

Beispiel 1 wurde mit der Abweichung wiederholt, daß anstelle von SV40-T-Antigen diesmal IL-2 solubilisiert wurde, das mit Hilfe von E.-coli-Zellen, die das Plasmid ptac4 enthielten, produziert wurde. Abweichend von Beispiel 1 wurde die NaCl-Konzentration im Puffer B auf 500 mM NaCl erhöht. Die Aktivität des solubilisierten IL-2 wurde durch einen T-Zellen-Wachstumstest bestätigt (siehe Tabelle 3).

**Tabelle 3**

| Aktivitätstest für IL-2 mit CL3-T-Zellen (Isolierung des IL-2 nach Beispiel 3) | | |
|---|---|---|
| Fraktion | Thymidin-Einbau (cpm) | IL2-Aktivität (%) |
| Gesamtlysat | 2617,0 | 100 |
| Überstand der Bindung an Q-Sepharose-FF® | 178,0 | 7 |
| Eluat des Lysats von Q-Sepharose-FF® | 5379,5 | 205 |

### Beispiel 4: Solubilisierung von Interleukin 2 (IL-2)

Es wurde Beispiel 3 mit der Ausnahme wiederholt, daß nach dem Aufschluß der Zellen 15 min lang bei 4 °C zentrifugiert wurde, wobei einmal der Überstand und das andere Mal der im Puffer A resuspendierte Niederschlag mit dem Ionenaustauscher behandelt wurde. Die Aktivität des solubilisierten Proteins wurde wie in Beispiel 3 bestätigt (siehe Tabelle 4).

**Tabelle 4**

| Aktivitätstest für IL-2 mit CL3-T-Zellen (Isolierung des IL-2 nach Beispiel 4) | | |
|---|---|---|
| Fraktion | Thymidin-Einbau (cpm) | IL2-Aktivität (%) |
| Gesamtlysat | 2617,0 | 100 |
| Eluat des Überstandes von Q-Sepharose-FF® | 2583,0 | 99 |
| Eluat des Pellets von Q-Sepharose-FF® | 2808,5 | 107 |

### Beispiel 5: Solubilisierung von v-myb

Einschlußkörper des retroviralen Oncoproteins v-myb, die mit Hilfe von E. coli HB101 (unter trp-Kontrolle; pVM2028) gebildet worden waren, ließen sich mit S-Sepharose-Fast-Flow® (S-Sepharose-FF®) solubilisieren, ohne daß eine Adsorption am Kationenaustauscher beobachtet wurde.

### Beispiel 6: Solubilisierung von T260

In Analogie zu Beispiel 5 ließen sich Einschlußkörper des T-Antigen-Peptids T260 mit S-Sepharose® solubilisieren.

### Beispiele 7 und 8: Solubilisierung von 3d^{rif}

In Analogie zu Beispiel 1 ließen sich Einschlußkörper eines Membranproteins, das für eine Rifampicin-Resistenz neuen Typs verantwortlich ist, mit Q-Sepharose® und auch Phenyl-Sepharose® solubilisieren.

### Liste von Abkürzungen

- IPTG =: Isopropyl-β-D-thiogalactopyranosid
- EDTA =: Ethylendiamintetraessigsäure
- DTT =: Dithiothreitol
- PMSF =: Phenylmethylsulfonylfluorid
- cpm =: counts pro min

### Liste von Lebendmaterial

- E. coli JM103: vgl. Nucleic Acids Res., 9 (1981) 309-321
- E. coli HB101: vgl. J. Mol. Biol., 41 (1969) 459-472
- CL3-T (Zellen): vgl. Biochemistry, 22 (1983) 251-255
- pVM2028 (Plasmid): vgl. EMBO J., 6 (1987) 2719-2725
- pTh (Plasmid): vgl. J. Virol., 62 (1988) 1999-2006
- ptac4 (Plasmid): Hoechst AG (Geschenk)
- trp: üblicher käuflicher Promotor

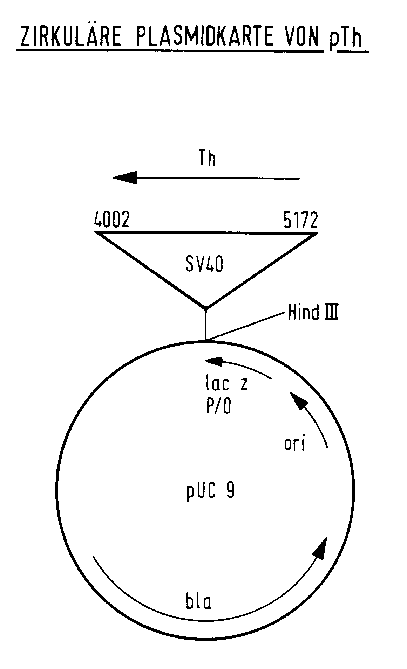

## Patentansprüche

1. Verfahren zur Solubilisierung von unlöslichem, von kultivierten Zellen exprimiertem Protein, dadurch **gekennzeichnet,** daß man das unlösliche Protein mit einem Ionenaustauscher zusammenbringt und danach vom Ionenaustauscher trennt.

2. Verfahren nach Anspruch 1, dadurch **gekennzeichnet,** daß man ein von E. coli exprimiertes Protein solubilisiert.

3. Verfahren nach Anspruch 1 oder 2, dadurch **gekennzeichnet,** daß man das Protein mit dem Ionenaustauscher in der Kulturbrühe in Gegenwart der aufgeschlossenen Zellen und der Zelltrümmer zusammenbringt.

4. Verfahren nach Anspruch 1 oder 2, dadurch **gekennzeichnet,** daß man das Protein mit dem Ionenaustaucher nach Abtrennung der aufgeschlossenen Zellen und der Zelltrümmer zusammenbringt.

5. Verfahren nach einem der vorhergehenden Ansprüche, dadurch **gekennzeichnet,** daß man den Ionenaustauscher in körniger Form verwendet.

6. Verfahren nach einem der vorhergehenden Ansprüche, dadurch **gekennzeichnet,** daß man als Ionenaustauscher einen Anionenaustauscher verwendet, insbesondere Q-Sepharose®.

7. Verfahren nach einem der vorhergehenden Ansprüche, dadurch **gekennzeichnet,** daß man als Ionenaustauscher einen Kationenaustauscher verwendet, insbesondere S-Sepharose®.

8. Verfahren nach einem der vorhergehenden Ansprüche, dadurch **gekennzeichnet,** daß man das Protein durch Eluieren vom Ionenaustauscher abtrennt.

## Claims

1. Method for the solubilisation of insoluble protein expressed by cultivated cells, characterised in that the insoluble protein is contacted with an ion exchanger and then separated from the ion exchanger.

2. Method according to Claim 1, characterised in that a protein expressed by E. coli is solubilised.

3. Method according to Claim 1 or 2, characterised in that the protein is contacted with the ion exchanger in the culture broth in the presence of the disrupted cells and of the cell detritus.

4. Method according to Claim 1 or 2, characterised in that the protein is contacted with the ion exchanger after removal of the disrupted cells and of the cell detritus.

5. Method according to one of the preceding claims, characterised in that the ion exchanger is used in the form of granules.

6. Method according to one of the preceding claims, characterised in that an anion exchanger is used as ion exchanger, especially Q-Sepharose®.

7. Method according to one of the preceding claims, characterised in that a cation exchanger is used as ion exchanger, especially S-Sepharose®.

8. Method according to one of the preceding claims, characterised in that the protein is separated from the ion exchanger by elution.

## Revendications

1. Procédé pour solubiliser de la protéine insoluble, exprimée par des cellules cultivées, procédé caractérisé en ce qu'on met en contact la protéine insoluble avec un échangeur d'ions et l'on sépare ensuite l'échangeur d'ions.

2. Procédé selon la revendication 1, caractérisé en ce qu'on solubilise une protéine exprimée par E. coli.

3. Procédé selon la revendication 1 ou 2, caractérisé en ce qu'on met en contact la protéine avec l'échangeur d'ions dans le bouillon de culture, en présence des cellules ouvertes ou éclatées et des débris de cellules.

4. Procédé selon la revendication 1 ou 2, caractérisé en ce qu on met en contact la protéine avec l'échangeur d'ions, après avoir séparé les cellules éclatées et les débris cellulaires.

5. Procédé selon l'une des revendications précédentes, caractérisé en ce qu'on utilise l'échangeur d'ions sous forme grenue .

6. Procédé selon l'une des revendications précédentes, caractérisé en ce qu'on utilise comme échangeur d'ions un échangeur d'anions, notamment "Q-Sépharose"®

7. Procédé selon l'une des revendications précédentes, caractérisé en ce qu'on utilise comme échangeur d'ions un échangeur de cations, en particulier "S-Sépharose"®.

8. Procédé selon l'une des revendications précédentes, caractérisé en ce qu'on sépare la protéine par élution de l'échangeur d'ions.
